# FASCICULE DE BREVET EUROPEEN

(11) **EP 1 061 887 B1**
(45) Date de publication et mention de la délivrance du brevet: **24.05.2006**
(21) Numéro de dépôt: 00900577.8
(22) Date de dépôt: 14.01.2000
(51) Int. Cl.: A61K 31/66, A61K 35/30, A23L 1/30, A23L 1/302, A23J 7/00, A61K 31/20

(54) **NOUVELLE UTILISATION DE PHOSPHOLIPIDES D'ORIGINE VEGETALE ET ANIMALE EN THERAPEUTIQUE NUTRITIONNELLE**
NEUE VERWENDUNG VON PHOSPHOLIDEN PFLANZLICHEN UND TIERISCHEN URSPRUNGS IN NAHRUNGSTHERAPIE
NOVEL USE OF PHOSPHOLIPIDS OF VEGETABLE AND ANIMAL ORIGIN IN NUTRITIONAL THERAPY

(30) Priorité: 14.01.1999 FR 9900335
(43) Date de publication de la demande: 27.12.2000
(73) Titulaire: Institut de Recherche Biologique S.A., 78340 Les Clayes-sous-Bois (FR)
(72) Inventeur: PONROY, Yves, CH-1820 Montreux (CH)
(74) Mandataire: Burtin, Jean-François
(86) Numéro de dépôt international: PCT/FR2000/000069
(87) Numéro de publication internationale: WO 2000/041484

(56) Documents cités:
- EP-A- 0 342 795
- EP-A- 0 661 056
- EP-B- 0 502 765
- WO-A-96/00016
- WO-A-98/16216
- WO-A-98/48788
- WO-A-98/50052
- FR-A- 2 721 516
- FR-A- 2 749 133
- US-A- 5 709 888
- HIBBELN ET AL.: "Dietary polyunsaturated fatty acids and depression: when cholesterol does not satisfy" AM. J. CLIN. NUTR., vol. 62, 1995, pages 1-9, XP002071081 cité dans la demande
- PEET ET AL.: "Depletion of omega-3 fatty acid levels in red blood cell membranes of depressive patients" BIOL. PSYCHIATRY, vol. 43, no. 5, 1 mars 1998 (1998-03-01), pages 315-319, XP002071083 cité dans la demande
- HIBBELN ET AL.: "Fish consumption may predict a lower prevalence of major depression : a cross-national analysis" WORLD REV. NUTR. DIET., vol. 83, 1998, pages 226-227, XP002118773
- BEHAN P O ET AL: "Effect of high doses of essential fatty acids on the postviral fatigue syndrome." ACTA NEUROLOGICA SCANDINAVICA, (1990 SEP) 82 (3) 209-16. , XP001028124
- UMEZAWA ET AL.: "Effect of dietary n-3 versus n-6 polyunsaturated fatty acids on learning and memory in the senescence-accelerated mouse (SAM)" KOSHIEN DAIGAKU KIYO, vol. 24, no. a, 1996, pages 55-61, XP001027988
- STEVENS L.J. ET AL: "Omega - 3 fatty acids in boys with behavior, learning, and health problems." PHYSIOLOGY AND BEHAVIOR, (1996) 59/4-5 (915-920). , XP001027872
- GOZZO S ET AL: "EFFECTS OF DIETARY PHOSPHO LIPIDS AND ODD CHAIN FATTY-ACID ON THE BEHAVIORAL MATURATION OF MICE." FOOD CHEM TOXICOL, (1982) 20 (2), 153-158. , XP001028520
- AYRE K J ET AL: "Effects of changes in dietary fatty acids on isolated skeletal muscle functions in rats." JOURNAL OF APPLIED PHYSIOLOGY, (1996 FEB) 80 (2) 464-71. , XP001027948
- DATABASE WPI Week 199002 Derwent Publications Ltd., London, GB; AN 1990-011028 XP002182844 & JP 01 290625 A (NIPPON OILS AND FATS CO LTD), 22 novembre 1989 (1989-11-22)
- DATABASE WPI Week 199722 Derwent Publications Ltd., London, GB; AN 1997-236803 XP002182845 & CN 1 099 618 A (FURENDE HEALTH CARE NEW TECH. DEV. CO. BEIJ.), 8 mars 1995 (1995-03-08)
- DATABASE EPODOC [en ligne] EUROPEAN PATENT OFFICE, THE HAGUE, NL; XP002182842 & CN 1 194 141 A (ZHANG XINGCHEN) 30 septembre 1998 (1998-09-30)
- CONNOR WE ET AL: "Dietary effects on brain fatty acid composition: the reversibility of n-3 fatty acid deficiency and turnover of docosahexaenoic acid in the brain, erythrocytes, and plasma of rhesus monkeys." JOURNAL OF LIPID RESEARCH, FEB 1990, 31;(2); P237-47, XP001041046 UNITED STATES
- AMAN MG ET AL: "The effects of essential fatty acid supplementation by Efamol in hyperactive children." JOURNAL OF ABNORMAL CHILD PSYCHOLOGY, MAR 1987, 15;(1); P75-90, XP001041059 UNITED STATES
- COLQUHOUN I ET AL: "A lack of essential fatty acids as a possible cause of hyperactivity in children." MEDICAL HYPOTHESES, MAY 1981, 7;(5); P673-9, XP001041058 ENGLAND
- MITCHELL EA ET AL: "Clinical characteristics and serum essential fatty acid levels in hyperactive children." CLINICAL PEDIATRICS, AUG 1987, 26;(8); P406-11, XP001041057 UNITED STATES
- DATABASE MEDLINE [en ligne] Dialog AN= 9511304, XP002182843 & LAMBERT EV ET AL: "Enhanced endurance in trained cyclists during moderate intensity exercise following 2 weeks adaptation to a high fat diet." EUROPEAN JOURNAL OF APPLIED PHYSIOLOGY AND OCCUPATIONAL PHYSIOLOGY, 1994, 69;(4); P287-93, GERMANY
- MUOIO DM ET AL: "Effect of dietary fat on metabolic adjustments to maximal VO2 and endurance in runners." MEDICINE AND SCIENCE IN SPORTS AND EXERCISE, JAN 1994, 26;(1); P81-8, XP001041038 UNITED STATES

## Description

La présente invention se rapporte au domaine de la biologie et plus particulièrement à celui de la thérapeutique et de la nutrition.

La présente invention a plus particulièrement pour objet l'utilisation de phospholipides d'origine naturelle sous forme de préparations diététiques ou sous forme de compositions pharmaceutiques ayant une action sur l'humeur, sur la fatigue nerveuse et sur l'agressivité.

L'invention a spécifiquement pour objet l'utilisation de nouvelles préparations ou compositions visant à combattre une carence en acides gras du type n-3 qui sont caractérisées en ce qu'elles contiennent comme ingrédient actif des phospholipides d'origine animale, riches en acide docosahexanénoïque (DHA), seuls ou additionnés d'huiles végétales ou animales riches en acides gras de la série n-3, en association ou en mélange avec un excipient ou un véhicule inerte non toxique.

Plusieurs études épidémiologiques ont contribué à démontrer que la fatigue nerveuse, l'agressivité et l'humeur dépressive sont associées à une carence en acide gras de la série n-3, c'est-à-dire principalement en DHA et en son précurseur, l'acide alpha linolénique (18:3 n-3) (J. R. HIBBELN, Dietary polyunsaturated fatty acids and depression, Am. J. Clin. Nutr. 62, 1-9 [1995]).

Au contraire, un excès d'acides gras de la série n-6, représentés par l'acide linoléïque (18:2 n-6) présent dans les huiles végétales, utilisées en abondance chez les occidentaux (huile d'arachide, de tournesol ou de maïs), peut être considéré comme étant à l'origine d'une grande vulnérabilité aux stress de la vie et à la dépression. Ainsi, les Sociétés qui consomment de grandes quantités de poissons riches en DHA et en acides gras n-3 semblent avoir un plus faible taux de dépression. Dans les villages de pêcheurs du Japon, la dépression est quasiment inexistante (K. HASEGAWA : The epidemiological study of depression in later life, J. Affect. Disord S1, p.1-6 [1985]).

Une autre étude a montré que la prévalence de la dépression est 10 fois plus forte aux U.S.A. et en Europe qu'à Taiwan. Ceci peut s'expliquer par le fait que, d'un côté, les Occidentaux consomment peu de poisson et une grande quantité d'huiles végétales riches en acide gras n-6 et carencées en n-3 (huiles de maïs, tournesol, arachide) et que, de l'autre, les Chinois de Taiwan consomment du poisson et de l'huile de soja riche en n-3 : The changing rate of major depression: cross national comparaison, JAMA 268, 1098-3105 (1992).

Cette explication se trouve renforcée par la constatation que la dépression du post-partum est accompagnée d'une baisse importante du taux de DHA plasmatique (M. GITLIN : Psychiatric syndrome liked to reproductive function in women, Am. J. Psychiatry 46, 1413-1422 [1989]).

De même, des auteurs ont mis en évidence le fait que les globules rouges de patients dépressifs ont une faible teneur en acides gras de la série n-3 (M. PEET : Depletion of Omega 3 fatty acid levels in red blood cell membranes of depressive patients, Biol. Psychiatry 43, 315-319 [1998]).

D'un point de vue métabolique, il a été montré chez l'animal qu'une carence en acides gras n-3 entraîne diverses perturbations au niveau des neuro-transmetteurs dopaminergiques et serotoninergiques, ce qui permettrait d'expliquer les troubles comportementaux qui en résultent : S. DELION : Chronic dietary alpha linolenic acid deficiency alters dopaminergie and serotoninergie neurotransmission in rats, J. Nutr. 124, 2466-2476 (1994).

Un apport nutritionnel d'acides gras n-3 sous forme de phospholipides d'animaux, riches en DHA, peut donc constituer une des meilleures approches pour un traitement physiologique de la dépression (M. MAES : Fatty acids, Cytokine and Major depression, Biol. Psychiatry 43, 313-314 [1998]).

On a déjà décrit dans le brevet français 2.721.516 de nouvelles utilisations d'un complexe à base de phospholipides cérébraux extraits de cerveaux de porc en thérapeutique et dans l'alimentation, en vue de compenser ou de ralentir les phénomènes de vieillissement, et plus précisément la détérioration mentale, l'hypoxie cérébrale et le vieillissement neurologique.

Dans ce brevet, on a divulgué en particulier l'utilité de phosphoglycérides liés à deux chaînes d'acides gras, soit par une fonction ester (phosphoglycérides) soit par une fonction éther (plasmalogènes).

On a montré que les phospholipides cérébraux ont en outre la particularité d'être liés à des acides gras dont la distribution est tout à fait spécifique et bien distincte de celle d'autres phospholipides, comme notamment ceux de soja.

On a trouvé maintenant que les phospholipides cérébraux, à des doses bien inférieures à celles décrites précédemment, trouvaient de nouvelles utilisations notamment dans le domaine des troubles du comportement, dans le domaine des troubles de l'humeur et dans le domaine des troubles de l'affectivité.

Ces utilisations ne s'adressent donc plus aux personnes âgées, mais plutôt aux personnes d'âge moyen (de 30 à 60 ans et, en particulier, de 40 à 50 ans) ayant des troubles de l'humeur et à des sujets jeunes présentant un syndrome d'hyperactivité. Dans ces conditions, les nouvelles compositions à base de phospholipides cérébraux n'agissent pas sur les syndromes de détérioration intellectuelle ou nerveuse, mais bien plutôt sur l'équilibre psychologique. Les compositions selon l'invention peuvent être additionnées d'autres sources d'acides gras de la série n-3, comme par exemple des huiles végétales ou animales riches en acides gras de la série n-3, comme l'huile de lin, l'huile de noix, l'huile de colza, l'huile de bourrache, l'huile d'onagre ou l'huile de poisson comme l'huile de thon, l'huile de sardine, l'huile de flétan ou l'huile de cabillaud.

Les compositions selon l'invention peuvent être additionnées de vitamines et en particulier de vitamines du groupe B, à savoir la vitamine B1, la vitamine B2, la vitamine B6, la vitamine F, l'acide folique, la nicotinamide, l'acide panthoténique, leurs dérivés ou leurs sels.
Les compositions selon l'invention renferment de 10 à 300 mg de phospholipides spécifiques du cerveau. Elles sont administrées de 1 à 4 fois par jour par voie digestive. Elles ne manifestent ni toxicité ni phénomène d'intolérance.
Les compositions contiennent en outre des excipients ou des diluants adaptés à l'administration digestive sous forme de comprimés nus ou enrobés, de comprimés à mâcher, de comprimés à sucer, de pilules, de cachets, de gélules, de capsules ou similaires, de granules, de poudres aromatisées ou non.
Dans les capsules molles, les phospholipides de cerveau peuvent être dispersés ou mis en suspension dans de l'huile de poisson.

Les excipients ou diluants appropriés pour ces voies peuvent être des produits minéraux inertes, comme par exemple le carbonate de calcium, le phosphate tricalcique, le phosphate de magnésium, l'alumine, la silice colloïdale, le kaolin, les argiles, le silicate d'aluminium, le silicate de calcium ou l'oxyde de fer pour la voie orale, ou l'eau ou les liquides aqueux ou les huiles pour les formes liquides.

Les supports inertes peuvent être de nature organique comme les amidons, les dextrines, le lactose, la cellulose, les dérivés synthétiques de cellulose, les alginates, les carraghénates, les caséinates, les acides gras, les cires ou les résines.

Les supports pour préparations diététiques peuvent avoir une valeur alimentaire ou constituer un apport calorique comme la caséine, la poudre de lait, le dextrose, les maltodextrines. Les compositions peuvent être aromatisées avec par exemple de la vanilline ou du maltol, ou édulcorées avec de l'aspartame ou de l'acesulfame.

### EXEMPLE I

### Gélules de phospholipides cérébraux de porc

- phospholipides d'origine cérébrale 300 g
- lactose 100 g
- phosphate tricalcique 1 40 g
- caséinate de calcium 25 g
- stéarate de magnésium 5 g
pour 1 000 gélules

### EXEMPLE II

### Dragées de phospholipides cérébraux de porc

- phospholipides d'origine cérébrale 10 g
- vitamine B1 0,5 g
- vitamine B6 0,5 g
- carbonate de magnésium 10 g
- carbonate de calcium 100 g
- lactose 100 g
- amidon de blé 50 g
- stéarate de calcium 20 g
pour 1 000 dragées

### EXEMPLE III

### Capsules molles de phospholipides cérébraux de porc

- PLC 100 g
- Huile de poisson 100 g
pour 1 000 capsules molles

### EXEMPLE IV

### Etude clinique des compositions selon l'invention

On a réalisé, à la suite de constatations préliminaires, une étude clinique contrôlée en double aveugle contre placebo chez des sujets se plaignant de fatigue chronique et d'asthénie avec troubles de l'humeur. L'étude a été multicentrique sur 2 groupes homogènes d'adultes âgés de 30 à 60 ans. La durée de l'expérimentation a été de deux mois avec soit 4 dragées par jour d'un produit actif contenant uniquement 10 mg de phospholipides spécifiques de cerveau, par dragée, soit 4 dragées par jour d'un placebo.

| **Groupe expérimental** | **Groupe témoin** |
|---|---|
| 67 sujets | 65 sujets |
| 47 femmes, 20 hommes | 38 femmes, 27 hommes |
| âge moyen : 46,7 années | âge moyen : 44,6 années |
| poids moyen : 65,08 kg | poids moyen : 64,7 kg |
| niveau culturel : 1,63 | niveau culturel : 1,85 |
| (1 à 3) | (1 à 3) |

On a noté la même répartition, dans les deux lots, de l'intensité et de la nature des troubles répartis en : troubles du comportement, de l'humeur, de l'affectivité et des fonctions mentales.
Ces critères subjectifs ont donné lieu à une évaluation conjointe entre le patient et le médecin grâce à un questionnaire. L'analyse symptomatique des résultats obtenus se regroupe en 5 rubriques que l'on peut résumer dans le tableau ci-dessous.

### Pourcentage des critères améliorés

| | **Groupe expérimental (phospholipides)** | **Groupe témoin (placebo)** |
|---|---|---|
| Fonction mentale (tonus intellectuel) | 77,2 % | 32 % |
| Comportement (fatigue, dynamisme) | 80,3 % | 43,2 % |
| Humeur (dépressive) | 74,2 % | 52,6 % |
| Affectivité (repli sur soi, désinsertion) | 75 % | 41,9 % |
| Troubles neurologiques mineurs (sommeil, vertige) | 76 % | 29,7 % |

Tous ces résultats sont significatifs. L'ensemble de ces résultats met en évidence une amélioration statistiquement significative dans le groupe phospholipides spécifiques du cerveau, riches en DHA par rapport au groupe placebo.
La tolérance a été très bonne.

## Revendications

1. Utilisation des phospholipides d'origine animale, riches en acides gras non saturés, de la série n-3, en vue de la réalisation d'une préparation diététique et/ou pharmaceutique appropriée pour améliorer ou restaurer l'humeur.

2. Utilisation selon la revendication 1, **caractérisée en ce que** les préparations contiennent exclusivement comme phospholipides d'origine animale des phospholipides de cerveaux de porc.

3. Utilisation selon l'une des revendications 1 à 2 dans laquelle les phospholipides d'origine animale sont additionnés d'huiles végétales riches en acides gras non saturés de la série n-3 choisies parmi l'huile de noix, l'huile de soja, l'huile de colza, l'huile de lin, l'huile de bourrache et l'huile d'onagre.

4. Utilisation selon l'une des revendications 1 à 3 dans laquelle les phospholipides d'origine animale sont additionnés d'huile de poisson riche en acides gras non saturés de la série n-3.

5. Utilisation selon l'une des revendications 1 à 4 dans laquelle la préparation contient en outre des vitamines.

6. Utilisation selon la revendication 5 dans laquelle la préparation contient des vitamines du groupe B.

7. Utilisation selon l'une des revendications 1 à 6 dans laquelle la préparation est additionnée de diluants ou d'excipients ou de supports adaptés à l'administration digestive.

8. Utilisation selon l'une des revendications 1 à 7 dans laquelle la préparation renferme de 10 à 300 mg de phospholipides de cerveau par prise unitaire.

9. Utilisation selon l'une des revendications 1 à 8 dans laquelle les préparations diététiques contiennent en outre des supports ayant une valeur alimentaire ou constituant un apport calorique.

## Claims

1. Use of phospholipids of animal origin, rich in unsaturated fatty acids stemming from the n-3 serie, in view of the production of a diabetic and/or pharmaceutical composition suitable for improving or restoring the humour.

2. Use according to claim 1, wherein the compositions solely contain as the phospholipids of animal origin, phospholipids from pig brains.

3. Use according to one of claims 1 to 2 wherein the phospholipids of animal origin are added to vegetal oils rich in unsaturated fatty acids of the n- 3 serie selected from the group consisting of nut oil, soja oil, colza oil, linssed oil, borage oil and evening primrose oil.

4. Use according to one of the claims 1 to 3, wherein the phospholipids of animal origin are added to fish oil rich in unsaturated fatty acids of the n-3 serie.

5. Use according to one of the claims 1 to 4, wherein the composition further contains vitamins.

6. Use according to claim 5 wherein the composition contains vitamins of the B group.

7. Use according to one of the claims 1 to 6, wherein the composition is added to diluents or carriers or excipients suitable for the digestive administration.

8. Use according to one of the claims 1 to 7 wherein the composition contains from 10 to 300 mg of brain phospholipids per unit dosage.

9. Use according to one of the claims 1 to 8, wherein the dietetic compositions further contain some carriers having an alimentary value or constituting a calorigenic supply.

## Patentansprüche

1. Verwendung von Phospholipiden, die tierischen Ursprungs und reich an nicht gesättigten Fettsäuren sind, aus der Reihe n-3, im Hinblick auf die Herstellung eines diätetischen und/oder pharmazeutischen Präparats, das geeignet ist zum Verbessern oder Wiederherstellen des Gernütszustandes.

2. Verwendung nach Anspruch 1, **dadurch gekennzeichnet, dass** die Präparate als Phospholipide tierischen Ursprungs ausschließlich Phospholipide aus Schweinehirn beinhalten.

3. Verwendung nach einem der Ansprüche 1 oder 2, wobei die Phospholipide tierischen Ursprungs pflanzlichen Ölen zugegeben werden, die reich an nicht gesättigten Fettsäuren der Reihe n-3 sind, ausgewählt aus Nussöl, Sojaöl, Rapsöl, Leinöl, Borretschöl oder Nachtkerzenöl.

4. Verwendung nach einem der Ansprüche 1 bis 3, wobei die Phospholipide tierischen Ursprungs Fischöl beigegeben werden, das reich an nicht gesättigten Fettsäuren der Reihe n-3 ist.

5. Verwendung nach einem der Ansprüche 1 bis 4, wobei das Präparat unter anderem Vitamine enthält.

6. Verwendung nach Anspruch 5, wobei das Präparat Vitamine der Gruppe B enthält.

7. Verwendung nach einem der Ansprüche 1 bis 6, wobei das Präparat Verdünnungsmitteln oder Grundmassen oder Trägersubstanzen mit verdauungsfördernder Wirkung zugegeben wird.

8. Verwendung nach einem der Ansprüche 1 bis 7, wobei das Präparat zwischen 10 und 300 mg Phospholipide aus einmaliger Gehirnentnahme umfasst.

9. Verwendung nach einem der Ansprüche 1 bis 8, wobei die diätetischen Präparate unter anderem Träger umfassen, die einen Nährwert haben oder einen kalorischen Beitrag beinhalten.
